(19) Europäisches Patentamt
European Patent Office
Office européen des brevets

(11) Veröffentlichungsnummer: **0 040 685**
**A2**

# (12) EUROPÄISCHE PATENTANMELDUNG

(21) Anmeldenummer: **81102393.6**

(22) Anmeldetag: **30.03.81**

(51) Int. Cl.³: **A 61 K 31/48**, C 07 D 457/08

(30) Priorität: **15.04.80 DE 3014410**

(43) Veröffentlichungstag der Anmeldung: **02.12.81 Patentblatt 81/48**

(84) Benannte Vertragsstaaten: **AT BE CH DE FR GB IT LI LU NL SE**

(71) Anmelder: **Meditest Institut für medizinisch-pharmazeutische Untersuchungen GmbH & Co. KG, D-7958 Laupheim (DE)**

(72) Erfinder: **Lange, Fritz-Walter, Königswieser Strasse 26, D-8035 Gauting (DE)**
Erfinder: **Müller, Jens, Königswieser Strasse 26, D-8035 Gauting (DE)**

(74) Vertreter: **Berg, Wilheim, Dr. et al, Dr. Berg, Dipl.-Ing. Stapf, Dipl.-Ing. Schwabe, Dr. Dr. Sandmair Mauerkircherstrasse 45, D-8000 München 80 (DE)**

(54) **Lysergsäurederivate und deren pharmazeutische Zubereitungen.**

(57) Die Erfindung betrifft neue heterocyclische Verbindungen der allgemeinen Formel

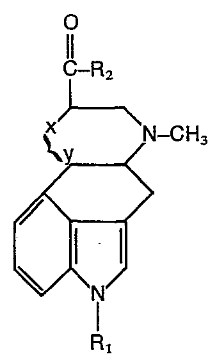

(I),

in der $R_1$ Wasserstoff oder die Methylgruppe, $\overset{\frown}{xy}$ die Gruppen $-CH_2$ oder $-CH$, $R_2$ die Gruppierung

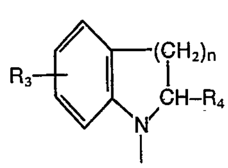

in der n = 1 oder 2, $R_3$ = Cl, Br, J, OH, $OR_4$, $NH_2$ oder $NO_2$ und $R_4$ = einen geradkettigen oder verzweigten $C_1$–$C_4$-Alkylrest bedeuten, sowie sie enthaltende pharmazeutische Zubereitungen.

EP 0 040 685 A2

0040685

Die Erfindung betrifft neue pharmakologisch wirksame heterocyclische Verbindungen der allgemeinen Formel I

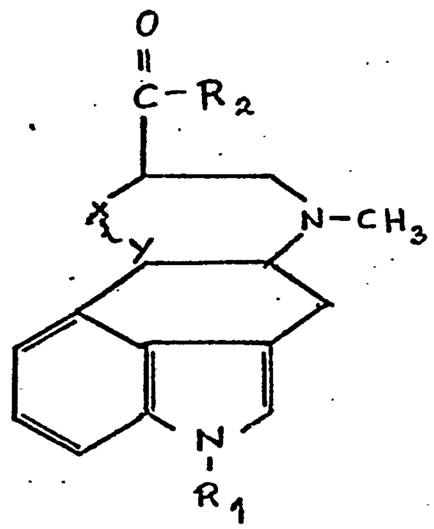

(I),

in der

$R_1$ Wasserstoff oder die Methylgruppe,

x~y die Gruppen $-CH_2$ oder -CH

$R_2$ die Gruppierung

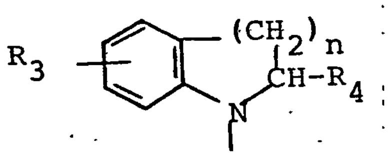

,

bedeutet, in der

n = 1 oder 2,

$R_3$ = Cl, Br, J, OH, $OR_4$, $NH_2$ oder $NO_2$ und

$R_4$ = ein geradkettiger oder verzweigter $C_1$-$C_4$-Alkylrest ist.

Bei den erfindungsgemäßen Verbindungen handelt es sich um neue Indolin- und Chinolinderivate der Lysergsäure und Dihydrolysergsäure, die gegebenenfalls in $R_1$ statt Wasserstoff die Methyl-

gruppe aufweisen können.

Es ist seit langem bekannt, daß sowohl die natürlich vorkommenden Mutterkornalkaloide als auch die halbsynthetischen Ergotalkaloide ein breites pharmakologisches Wirkungsspektrum haben.

Die in der Literatur beschriebenen halbsynthetischen Ergotalkaloide weisen eine relativ hohe Toxidität oder eine große Zahl unerwünschter, teils schädlicher Nebenwirkungen auf, was ihre pharmazeutische Verwendung erheblich beschränkt.

In Beispiel 13 der DE-OS 1 770 227 ist die Herstellung von d-Lysergsäure-2-amido-1-hydroxy-3-indolylpropan beschrieben, jedoch ist diese Substanz als mit Aminoalkoholen gebildet von den nachfolgend beschriebenen Verbindungen strukturell verschieden. Als pharmakologische Wirkung wird von den in der DE-OS 1 770 227 beschriebenen Substanzen lediglich ganz allgemein eine Antiserotoninwirkung angegeben. Mit den erfindungsgemäßen Verbindungen werden neue wertvolle Arzneimittel zur Verfügung gestellt, die spezifische Wirkungsrichtungen z.B. antidepressive Wirkung, Bradykardia-Wirkung, Inhibition der durch Collagen induzierten Plättchen-Aggregation und eine diuretische Wirkung bei gleichzeitig guter Verträglichkeit aufweisen.

Der Erfindung liegt die Aufgabe zugrunde, neue halbsynthetische Ergotalkaloide zur Verfügung zu stellen, die bei guter Verträglichkeit und geringer Toxidität spezifisch wirksam sind und eine eindeutige therapeutische Wirkung aufweisen.

Es wurde überraschend gefunden, daß man diesen Anforderungen voll entsprechende neue Derivate der Lysergsäure und der Dihydrolysergsäure erhält, wenn man Lysergsäure, Dihydrolysergsäure oder deren 1-Methylderivate mit N,N'-Carbodiimidazol in einem geeigneten Lösungsmittel, vorzugsweise Dimethylformamid, unter Erwärmen umsetzt und die entstandenen Imidazole mit einem Indolin- oder Chinolinderivat der Formel II

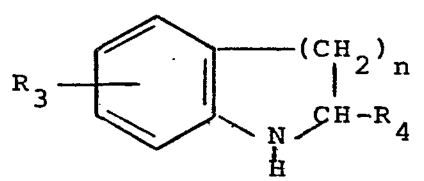

(II)

vorzugsweise bei Raumtemperatur zur Reaktion bringt, wobei n, $R_3$ und $R_4$ die gleiche Bedeutung wie in Formel I haben, und die neue Substanz nach bekannten Methoden aus dem Reaktionsgemisch isoliert, zur Reinigung aus einem geeigneten Lösungsmittel umkristallisiert oder nach einem bekannten säulenchromatographischen Verfahren in reiner Form gewinnt.

0040685

Beispiel 1

In einem 500 ml Dreihalskolben wird eine Mischung aus 200 ml Dimethylformamid, p.a., 1,35 g (5 mMol) Dihydrolysergsäure, wasserfrei, sowie 2,43 g (15mMol) N,N -Carbodiimidazol gegeben und 90 Min. unter Rühren auf 70° C erwärmt. Nach dem Abkühlen auf Raumtemperatur und nach Zugabe von 1,7 g (10 mMol) Indolin wird die Lösung 24 h bei Raumtemperatur aufbewahrt.

Anschließend wird das Lösungsmittel im Wasserstrahlvakuum bei max. 70° C Sumpftemperatur abdestilliert und zu dem Destillationsrückstand werden 200 ml Wasser, dem 2 ml Essigsäure zugesetzt worden sind, eingerührt. Nach weiteren 12 h Rührzeit wird die wässrige Phase von dem an der Kolbenwand haftenden Rückstand abgegossen. Dann wird der Kolbenrückstand nochmals mit 200 ml 1-proz-Ammoniakwasser 6 h lang durchgerührt und schließlich abgesaugt.

Das Reaktionsprodukt wird in 20 ml Methanol gelöst, die Lösung mit 1 g Aktivkohle behandelt, filtriert und das Filtrat auf 10 ml eingeengt. Dann wird das Konzentrat bis zur vollständigen Ausfällung mit Wasser versetzt. Sobald die anfangs schmierige Ausfällung kristallin geworden ist, wird sie abgesaugt, mit etwas Wasser nachgewaschen und getrocknet.

0040685

Ausbeute: 1,285 g

= 70 % der Theorie

Fp = 240 - 242° C

Nach dem gleichen Verfahren wie in Beispiel 1 werden die neuen Substanzen der Tabelle 1 hergestellt:

Tabelle 1

neue Derivate der Dihydrolysergsäure

| Beispiel Nr. | verwendetes Indolin- od. Chinolinderivat | erhaltenes neues Derivat der Dihydrolysergsäure $R_{13}$ | $R_{14}$ | n | Fp 0° C |
|---|---|---|---|---|---|
| 2 | 2-Methylindolin | H | $CH_3$ | 1 | 282-284 |
| 3 | 5-Chlorindolin | 5-Cl | H | 1 | Harz |
| 4 | 5-Nitroindolin | $5\text{-}NO_2$ | H | 1 | 88-90 |
| 5 | 6-Aminoindolin | $6\text{-}NH_2$ | H | 1 | 105-107 |
| 6 | Tetrahydrochinolin | H | H | 2 | 123-126 |

Beispiel 7

Herstellung von Lysergsäureindolid

In einem 500 ml Dreihalskolben wird eine Mischung aus 200 ml wasserfreiem Dimethylformamid, 1,35 g wasserfreier Lysergsäure sowie 2,43 g N,N'Carbonyldiimidazol 8 h lang unter Ausschluß von Licht gerührt. Dann werden 1,79 g Indolin zugegeben und das Rühren noch weitere 24 h fortgesetzt. Anschließend wird

das Lösungsmittel bei 1 Torr und höchstens 40°C Sumpftemperatur bis auf wenige ml abdestilliert und zu dem Destillationsrückstand werden 200 ml Wasser eingerührt. Nach einer Rührzeit von weiteren 12 h wird das ausgefallene feine Pulver abgesaugt, reichlich mit 2-proz. Ammoniakwasser gewaschen und getrocknet. Dieses Rohprodukt wird zur Reinigung in 30 ml Methanol gelöst, die Lösung mit 1 g Aktivkohle behandelt, filtriert, das Filtrat bis auf 5 ml eingeengt und das Konzentrat mit Wasser (200 ml) bis zur vollständigen Ausfällung versetzt. Bei längerem Stehen wird das anfänglich schmierige Produkt fest. Es wird abgesaugt, mit Wasser nachgewaschen und getrocknet (Vakuum, über $P_4O_{10}$).

Ausbeute: 1,1 g (62,6 % d. Theorie) Lysergsäureindolid.
$C_{24}H_{21}N_3O$

Die Substanz des Beispiels 1 zeigt antidepressive Eigenschaften bei geringer Toxizität.
Die Substanz des Beispiels 2 zeigt im Plättchen-Test bei der durch Collagen induzierten Aggregation eine deutliche Inhibition bei sehr niedriger Dosierung.
Die gleichen Eigenschaften zeigt die Verbindung des Beispiels 4, wobei noch zusätzlich eine deutliche Antihistaminwirkung erkennbar ist.

Die Verbindung des Beispiels 5 weist eine deutliche Bradykardia-Wirkung und milde diuretische Eigenschaften auf.

Nach dem gleichen Verfahren wie in Beispiel 7 sind auch neue Indolin- und Chinolinderivate der Lysergsäure herzustellen, wobei in der Verfahrensweise darauf zu achten ist, daß die Reaktionen stets unter Ausschluß von Licht und Luftsauerstoff erfolgen.

DR. BERG DIPL.-ING. STAPF
DIPL.-ING. SCHWABE DR. DR. SANDMAIR
PATENTANWÄLTE
Postfach 86 02 45 · 8000 München 86

0040685

Anwaltsakte: 50 146

Meditest - Institut für
medizinisch-pharmazeutische
Untersuchungen GmbH & Co.KG
Laupheim / Bundesrep. Deutschland

Patentansprüche

1. Heterocyclische Verbindungen der allgemeinen Formel

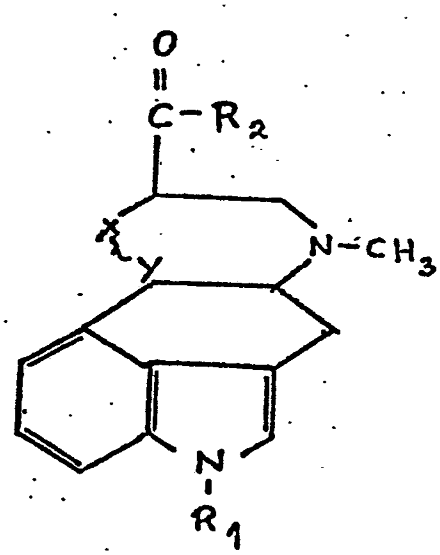

(I),

in der

$R_1$ Wasserstoff oder die Methylgruppe,

x~y die Gruppen $-CH_2$ oder -CH,

$R_2$ die Gruppierung

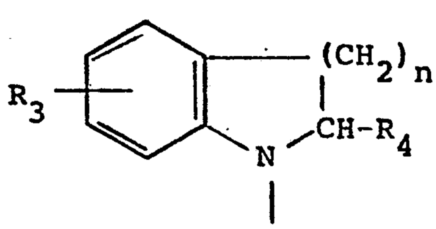

in der

n = 1 oder 2,

$R_3$ = Cl, Br, J, OH, $OR_4$, $NH_2$ oder $NO_2$ und

$R_4$ = einen geradkettigen oder verzweigten $C_1$-$C_4$-Alkylrest bedeuten.

2. Dihydrolysergsäureindolid, Lysergsäureindolid, Dihydrolysergsäure-(2)-methylindolid, Dihydrolysergsäure-(5)-chlorindolid, Lysergsäure-(5)-chlorindolid, Dihydrolysergsäure-(5)-nitroindolid, Dihydrolysergsäure-tetrahydrochinolid, Lysergsäure-tetrahydrochinolid, Dihydrolysergsäure-6-aminoindolid, Lysergsäure-(6)-aminoindolid.

3. Pharmazeutische Zubereitung enthaltend eine Verbindung gemäß den Ansprüchen 1 bis 2 zusammen mit üblichen physiologisch verträglichen Hilfs- und Trägerstoffen.